# EUROPEAN PATENT APPLICATION

(11) **EP 2 784 045 A1**
(43) Date of publication of application: **01.10.2014**
(21) Application number: 13161880.3
(22) Date of filing: 29.03.2013
(51) Int. Cl.: C04B 35/447, B22F 3/105, C04B 35/64

(54) **Selective laser sintering/melting process**

(71) Applicant: Osseomatrix, 91190 Gif-sur-Yvette (FR); ARMINES, 75272 Paris Cedex 06 (FR)
(72) Inventor: Colin, Christophe, 91003 Evry Cedex (FR); Bartout, Jean-Dominique, 91460 Marcoussis (FR); Shaker, Emmanuelle, 92260 Fontenay-aux-Roses (FR); Marchat, David, 42000 Saint-Etienne (FR); Nimal, Didier, 91190 Gif-sur-Yvette (FR)
(74) Representative: Icosa

(57) **Abstract**

The present invention relates to a process for manufacturing a three-dimensional article from a pulverulent substrate comprising at least a main substrate and at least an energy transferring vector, said process using at least one high energy source of a determined wavelength for sintering/melting the pulverulent substrate. The present invention relates also to a three-dimensional article manufactured from said process and to the layer manufacturing system.

## Description

### FIELD OF INVENTION

The present invention relates to the field of selective laser sintering/melting, and more especially to a three-dimensional article manufactured from a composite pulverulent substrate comprising particles in the form of a powder. This invention also relates to a manufacturing process of a three-dimensional article, said process involving bonding, i.e. fusing or sintering some of the particles of the substrate thanks to an energy source, preferably a laser. Advantageously, the manufacturing process of the invention is implemented layer-by-layer.

### BACKGROUND OF INVENTION

Selective laser sintering/melting is an additive manufacturing technique, i.e. a process, wherein an article is created by laying down successive layers of materials. This process is often referred as "layer manufacturing process". Since its creation in the Department of Mechanical Engineering at The University of Texas in the 1980s, great advances have been developed and selective laser sintering/melting processes are now widespread. These processes allow manufacturing complex three-dimensional shapes unattainable through molding, extrusion or other traditional processes.

The main feature of that kind of processes consists in sintering or melting powders with a high energy source, for example a laser, powder particles absorbing the energy of the laser. The selective laser sintering or melting process is a multi-physic process implementing both absorption of the laser energy and heat conduction, therefore leading to the sintering or melting of the particles of the powder.

### Technical issue

However, a technical issue remains in that selective laser sintering/melting processes of the prior art are restrained when the wavelength of the laser significantly differs from the absorption spectrum of the powder; in this case the powder is deemed "transparent" and the manufacture of three-dimensional articles is made impossible. Selective laser sintering/melting processes of the prior art require that the wavelength of the laser should exactly fit with the maximum of absorptivity of the powder.

The solution brought by the prior art to this problem is to enhance the amount of linear energy, for compensating the low absorption of the substrate. Enhancing the amount of linear energy is usually performed by enhancing the power of the laser and/or lowering the speed of the movement of the laser beam, and/or by using other sources of energy. These poor solutions result in a loss of productivity, in a poor quality of the final article due to insufficient bonding between the particles and cost-ineffectiveness.

These problems especially arise for ceramics powders such as calcium phosphate, particularly hydroxylapatite or tricalcium phosphate; for example pure white powder of hydroxyapatite is totally "transparent" to Nd-YAG laser (wavelength of 1064 nanometers), which is a common laser for industrial applications.

Concerning the sintering processes of the prior art in which the wavelength of the laser does not exactly fit with the wavelength of the maximum of absorptivity of the powder; WO2005/10541 discloses a method for the bonding of materials to give three dimensional objects, by means of a selective heating using electromagnetic energy, which is either non-coherent and/or non-chromatic and/or non-directed. The selectivity of the fusion is achieved by the application of an absorber via an inkjet process to defined partial regions of a layer of powder substrate; and subsequent heating of the absorber by means of electromagnetic energy. In the present invention the Applicant does not deposit an absorber on the partial regions to be fused or sintered but mixes the substrate with an energy transferring vector prior to the deposit of the substrate layer. Moreover the Applicant does not use a non-coherent, non-directed electromagnetic energy source but a directed laser. In WO2005/105412, the main issue is the implementation of a cost-effective process which does not require an expensive laser. In the present invention, one of the main issues is the implementation of a process which enables efficient densification of ceramic materials.

WO2012/164025 relates to a ceramic particle mixture containing, as components, a predominant portion by weight of frittable particles made of ceramic material and particles of at least one additive; said at least one additive being a dispersed absorbent material which has, for a laser beam emitted at a predetermined wavelength, a specific absorptivity that is greater than the absorptivity of the other components of the ceramic mixture and which drastically breaks down when gas is emitted in the presence of the laser beam. The process disclosed in WO2012/164025 is a subtractive process linked to the machining of ceramic material. A pulsed laser is used leading to a thermal choc in order to break down the ceramic material. This process needs a previous step for forming the ceramic material. In the present invention, the energy transferring vector is used for transferring the radiant energy of the laser into thermal energy in order to bond the ceramic material surrounding the energy transferring vector. It is an additive process, which does not need any previous step. Therefore the present invention differs clearly from the prior art.

It is an object of the present invention to address one or more drawbacks associated with the prior art and to provide a versatile process, allowing manufacturing articles from a high variety of pulverulent substrates, with no need of changing the laser equipment when the maximum of absorptivity of the pulverulent substrate does not exactly fit with the wavelength of the laser.

### SUMMARY

The foregoing objects are achieved by the implementation of a selective laser sintering/melting process for manufacturing an article from a pulverulent substrate comprising at least one main substrate and at least one energy transferring vector; said process implementing at least one high energy source.

In one embodiment, said at least one energy transferring vector comprises as chemical element: carbon, scandium, titanium, vanadium, chromium, manganese, iron, cobalt, nickel, copper, or zinc, or mixture thereof.

In one preferred embodiment, said energy transferring vector comprising carbon comprises carbon free or carbon no free or mixture thereof.

In one embodiment, said at least one main substrate comprises ceramic materials, metals, metals alloys, metals oxide, bioactive glasses, lead zirconate titanate, silicides, borides, carbides or mixture thereof.

In one preferred embodiment, said ceramic materials comprise calcium phosphate such as for example hydroxyapatite or tricalcium phosphate or mixture thereof.

In one embodiment, the process for manufacturing a three-dimensional article comprises the steps of:
a) Providing a layer of a pulverulent substrate, in a manufacturing chamber,
b) Optionally, controlling the temperature of the manufacturing chamber, or of the walls of the manufacturing chamber,
c) Selective laser sintering/melting of regions of the substrate layer by means of an energy source,
d) Optionally, repeating preceding steps a) to step c) until the desired article has been fashioned layer-by-layer.

In one embodiment, the amount of energy transferring vector is less than 5% (w/w) relative to the total weight of pulverulent substrate.

In one embodiment, the particle size of the main substrate ranges from 1 to 500 micrometers, preferably from 5 to 100 micrometers, more preferably from 10 to 25 micrometers.

In one embodiment, the particle size of the energy transferring vector ranges from 1 nanometer to 500 micrometers, preferably from 1 nanometer to 200 micrometers, more preferably from 10 nanometers to 100 nanometers.

In one embodiment, the at least one high energy source is a directed high energy source. In one preferred embodiment, the at least one high energy source is a laser, preferably a Nd - YAG laser, a CO₂ laser or a Er - YAG laser, more preferably a Nd - YAG laser.

One object of the present invention also relates to an article obtainable by the process of the present invention. In one preferred embodiment, the article is a biomedical device.

Another object of the present invention also relates to a system for manufacturing said article comprising:
- A computer file storing the description layer by layer of the three-dimensional article to manufacture,
- A directed high energy source for melting or sintering pulverulent substrate or pulverulent substrate layers, the directivity of the high energy source being based on the data of the computer file,
- A powder tank comprising an pulverulent substrate, which is comprising the main substrate and an energy transferring vector; during manufacture of the article, layers of pulverulent substrate from the powder tank are positioned under the high energy source.

### DETAILED DESCRIPTION

### Process

This invention thus relates to a selective laser sintering/melting process for manufacturing three-dimensional articles from a composite pulverulent substrate comprising at least one main substrate and at least one energy transferring vector, said process using at least one energy source of a determined wavelength for sintering/melting the pulverulent substrate.

In an embodiment, the process is an additive layer-by-layer manufacturing process, wherein a bed of particles is spread to form a layer of uniform thickness, and at least one energy source is directed to the layer, in order to fuse or sinter the particles.

The process for manufacturing a three-dimensional article of the invention comprises the steps of:
a) Providing a layer of a pulverulent substrate comprising at least one main substrate and at least one energy transferring vector, in a manufacturing chamber,
b) Optionally, controlling the temperature of the manufacturing chamber or of the walls of the manufacturing chamber,
c) Selective sintering/melting of regions of the pulverulent substrate layer by means of an energy source, preferably a laser of wavelength from 100 nanometers to 1 millimeter.

In an embodiment an energy transferring vector is added with, or mixed with, the main substrate prior to step a).

In another embodiment, step c) reads: selective sintering/melting of regions of the pulverulent substrate layer by means of a laser of wavelength from 100 nanometers to 1 millimeter.

In an embodiment, steps a) to c) are repeated until the desired article has been fashioned layer-by-layer.

In an embodiment, the manufacturing chamber is heated during the process between 300 and 1000°C, preferably between 300 and 900°C, more preferably between 400 and 800°C.

In an embodiment, the thickness of the layers of pulverulent substrate applied during step a) is from 0.001 millimeter to 10 millimeters, preferably from 0.005 millimeter to 1 millimeter, more preferably from 0.01 millimeter to 0.1 millimeter, even more preferably from 0.025 millimeter to 0.075 millimeter.

In an embodiment, the thickness of the layers of pulverulent substrate is adjustable between each deposited layers.

In one embodiment, the energy source settings, such as for instance the velocity and /or the power, are adjusted in order to limit the depth of the substrate altered by the energy source.

In an embodiment the settings of the energy source and the thickness of the layers of pulverulent substrate are adjusted in order to limit layers overlapping.

In an embodiment, the particle size of the pulverulent substrate ranges from 1 nanometer to 500 micrometers, preferably from 5 nanometers to 100 micrometers, more preferably from 10 nanometers to 25 micrometers.

In an embodiment, the wavelength of the energy source does not exactly fit with the wavelength of the maximum of absorptivity of the main substrate. In another embodiment, the wavelength of the energy source differs significantly from the wavelength of the maximum of absorptivity of the main substrate. In another embodiment, the main substrate is transparent to the energy source. A substrate is said to be transparent to an energy source if the substrate is incapable or insufficiently capable of absorbing the radiation from the energy source. Insufficiently means that absorption of radiation via an energy source cannot heat the substrate sufficiently to enable it to bond via fusion or sintering adjacent particles, or that the time needed for this is very long. So the main substrate does not absorb enough the energy of the energy source.

### Main substrate

In an embodiment of the invention, the main substrate has a maximum of absorptivity differing from the wavelength of the energy source, such that the manufacturing process is not as optimized (time, heat conduction) as it would be, should the absorption spectrum of the main substrate be well absorbing in the wavelength of the energy source.

The selective laser sintering/melting of a main substrate may occur in certain circumstances with an energy source having a wavelength which differs significantly from the maximum of absorptivity of the substrate. To achieve said sintering or melting the substrate must be slightly modified. A small amount of an energy transferring vector with an adapted absorption spectrum must be added to the main substrate. This energy transferring vector store sufficient energy from the energy source to sinter or melt the main substrate without another external energy supply. This energy transferring vector therefore leads to an efficient manufacturing as well as to an optimal densification of the article.

The forming of ceramics from powders necessarily generates porosity by fixing, in 3 dimensions, position and relationships of interparticle voids.

In one embodiment, the use of an energy transferring vector ensures a non-programed porosity of the manufactured device inferior to 30%, preferably inferior to 20%, preferably inferior to 10%, more preferably inferior to 5%, even more preferably inferior to 2%.

In one embodiment, the main substrate is in any form: liquid, solid, gas, powder..., preferably in a powder form.

In an embodiment, the particle size of the main substrate ranges from 1 to 500 micrometers, preferably from 5 to 100 micrometers, more preferably from 10 to 25 micrometers.

In an embodiment, the main substrate comprises calcium phosphate.

In one embodiment, the calcium phosphate comprises hydroxyapatite or tricalcium phosphate or mixture thereof; preferably with purity from 85 to 99.999%, more preferably with purity from 95 to 99.999%.

In on embodiment, the main substrate comprises ceramics, ceramics oxide, metals, metals alloys, metal oxide, silicides, borides, carbides, bioactive glasses, lead zirconate titanate, or mixtures thereof.

Ceramics may be preferably selected from alumina or alumina derivative (such as for example aluminosilicate); magnesia; zinc oxide; titanium oxide; barium titanate; silicates; tricalcium phosphate; apatite derivatives, preferably hydroxyapatite (including synthetic hydroxyapatite, more preferably substantially not degradable synthetic hydroxyapatite, carbonate-substituted hydroxyapatite, silicate-substituted hydroxyapatite); fluoroapatite or fluorohydroxyapatite or silicated apatite; zirconia, zirconia-toughened alumina (ZTA), alumina-toughened-zirconia (ATZ), ytria- zirconia (TZP), wallostonite; mixed oxide; or mixture thereof.

Metal and/or metal alloy are preferably selected from titanium; titanium alloys such as for example titanium-aluminum-vanadium; chrome-cobalt and alloys thereof, titanium-nickel alloys such as for example Nitinol, stainless steel or mixture thereof.

Bioactive glasses are recognized as materials suitable for bone repair or replacement. Bioglasses preferred in the present invention are silicate type materials composed of SiO₂ CaO and optionally Na₂O and/or P₂O₅. Preferred bioglasses are those as commercialized under the name "Bioglass45S5", or those having a composition as follows: 45-55% SiO₂, 10-25% (K₂0+Na₂0), 0-5% MgO; 10-25%CaO; 0-2%P₂O₅ and 0-1% B₂O₃ in weight, to the total weight of the bioglass. A preferred bioglass has the following composition: 45% SiO₂, 24.5% CaO and 24.5%Na₂O and 6%P₂O₅ in weight to the total weight of the bioglass. Another preferred bioglass has the following composition: 53% SiO₂, 11% K₂O and 6%Na₂O 5% MgO 22%CaO and 2%P₂O₅ and 1% B₂O₃ in weight, to the total weight of the bioglass.

Lead zirconate titanate (Pb[ZrₓTi₁₋ₓ]O₃ 0<x<1), also called PZT, is a ceramic perovskite material that shows a marked piezoelectric effect.

In one embodiment, the main substrate is a composite main substrate comprising at least two components, such as for example two components among those described hereabove.

### Eneray transferring vector

According to the invention, the energy transferring vector is well-absorbing in the wavelength of the energy source used in the process. Well-absorbing means that the energy received from the energy source and dissipated from the energy transferring vector is sufficient to bond the substrate adjacent to the energy transferring vector via fusion or sintering. By adding the energy transferring vector, the absorption of the energy source by the pulverulent substrate increases.

In one embodiment, the energy transferring vector presents, compared to the other components, an absorption differential above 0.2, preferably above 0.4, more preferably above 0.5. The absorption coefficient (A>=0) being defined as A=1-R, where R is the reflectivity coefficient. In the wavelength from 200 nanometers to 3 micrometers, the absorption coefficient of carbon may exceed 0.7.

In one embodiment, the energy transferring vector is in any form: liquid, solid, gas, preferably in a powder form.

Preferably, the particle size of the energy transferring vector ranges from 1 nanometer to 500 micrometers. More preferably, the energy transferring vector is in the form of nanoparticles of a size ranging from 1 nanometer to 200 micrometers, preferably from 10 nanometers to 100 nanometers.

In one embodiment, the amount of energy transferring vector is less than 5% (w/w) relative to the total weight of pulverulent substrate used in the process (main substrate + energy transferring vector), preferably from 0.01 to 2% (w/w), more preferably from 0.1 to 1% (w/w).

In one embodiment, the mass ratio of the energy transferring vector to the main substrate, in the pulverulent substrate, ranges from 0.000001 to 1, preferably from 0.00001 to 0.1, more preferably from 0.0001 to 0.2.

In one embodiment, the size ratio of the energy transferring vector to the main substrate, in the pulverulent substrate, ranges from 0.000001 to 1, preferably from 0.00001 to 0.1, more preferably from 0.0001 to 0.1.

In one embodiment, the energy transferring vector comprises carbon as chemical element. In one embodiment, the energy transferring vector comprising carbon may comprise carbon free or carbon no free or mixture thereof. In one embodiment, the energy transferring vector comprising carbon may be silicon carbide or carbon-such as for instance carbon black-; preferably with purity from 85 to 99.999%, more preferably with purity from 95 to 99.999%; or a mixture thereof.

In one embodiment, the energy transferring vector comprises as chemical element: carbon, scandium, titanium, vanadium, chromium, manganese, iron, cobalt, nickel, copper, or zinc, or mixture thereof.

### Energy source

In one embodiment the layer manufacturing process is performed thanks to at least one energy source, for example at least one laser.

In one embodiment, said high energy source(s) has a wavelength from 100 nanometers to 100 micrometers.

In one embodiment, said laser(s) used during the manufacturing process is a Nd - YAG laser and/or a CO2 laser and/or an Er - YAG laser, preferably a Nd-YAG laser (wavelength 1064 nanometers).

In one embodiment, different high energy sources are implemented for the pre-treatment and/or for the sintering/melting process and/or for the post-treatment; said high energy sources being of the same nature or of different nature.

In one embodiment, the same high energy source is used for the pre-treatment and/or for the sintering/melting process and/or for the post-treatment; said high energy source may be set differently for each step.

In one embodiment, the power of the energy source used during the manufacturing process ranges from 1 to 500 Watts, preferably from 5 to 300 Watts, more preferably from 10 to 150 Watts.

In one embodiment, the velocity of the energy source beam may range from 0.01 to 500 mm/s, preferably from 1 to 250mm/s, more preferably from 50 to 150 mm/s.

In one embodiment, the hatching space may range from 1 to 1000 micrometers, preferably from 10 to 500 micrometers, more preferably from 100 to 300 micrometers.

In one embodiment, the laser may be pulsed or continuous, preferably a continuous laser.

In one embodiment, the laser is the only energy source used during the process for sintering or melting the pulverulent substrate.

### Operating conditions

In one embodiment, the movement of the energy source beam or of the laser beam is controlled through a software controlled scanner system or any other means enabling the movement in x, y, z of the laser beam that a person skilled in the art would find appropriate.

In one embodiment, the manufacturing process is realized under argon atmosphere. In another embodiment, the layer manufacturing process is realized under regular (air) atmosphere conditions.

In one embodiment, the temperature of the manufacturing chamber is controlled.

In one embodiment, the pulverulent substrate used during the manufacturing process is prepared through wet process. In one embodiment, the solvent used during the wet process is an organic solvent, preferably methanol. In one embodiment, the pulverulent substrate is prepared by mixing 2/3, by volume, of organic solvent with 1/3, by volume, of a mixture comprising the main substrate and the energy transferring vector. The previous solution is then heated to 120°C until total evaporation. In another embodiment, the pulverulent substrate used during the manufacturing process is prepared through dry process. In one embodiment, the process for realizing the pulverulent substrate used during the manufacturing process is a 1, 2, 4, 6, 12, 24, or 48 hours process, more preferably a 24 hours process. Accordingly, the process of the invention may include a prior step, where the pulverulent substrate is prepared via a wet or a dry manufacturing. In an embodiment, the energy transferring vector forms with the main substrate an intimate mixture. In an embodiment, the pulverulent substrate is screened before to be used for the sintering/melting process, in order to remove particles larger than 500 micrometers, preferably larger than 100 micrometers, more preferably larger than 50 micrometers, even more preferably larger than 25 micrometers.

In one embodiment the settings implemented for an optimal manufacturing process are the following:
- Pre-treatment of the support as disclosed hereafter,
- Setting up the laser: power, velocity, hatching space, etc.,
- Setting up the pulverulent substrate layer settings: quantity of powder, etc.

In one embodiment, the pulverulent substrate may be pre-treated by heating prior to the layering step, at a temperature of 100°C to 1500°C, preferably of 200 to 1200°C, more preferably of 500 to 1000°C.

In one embodiment, the article may be post-treated, for example to enhance mechanical properties or to partially remove the energy transferring vector.

Said post-treatment may be the combination of an increase of the temperature and of the pressure.

In one embodiment, the post-treatment is achieved at a temperature between 300°C and 3500°C, preferably between 500 to 2500°C, more preferably between 1000 and 1800°C, even more preferably between 1000 and 1200°C.

In one embodiment, the post-treatment comprises a hot isostatic pressing.

In one embodiment the post-treatment include at least one ramp and/or at least one plateau of temperature and/or of pressure.

In one embodiment the post-heating is achieved during at least 30 minutes, at least 1 hour, at least 2 hours, or at least 6 hours.

In one embodiment, the post-heating is achieved with a heating rate of 5°C/min, 10°C/min or 20°C/min.

### Layer manufacturing system

The invention also relates to a layer manufacturing system used for performing the process described hereabove.

In one embodiment, the layer manufacturing system for realizing three-dimensional article through selective laser sintering/melting comprises a computer file storing the description layer by layer of the three-dimensional article to manufacture.

In one embodiment, the high energy source is a directed high energy source, i.e. a high energy source with a predetermined trajectory. This predetermined trajectory is based on the computer file storing the description layer by layer of the article to manufacture. This programed trajectory may define voids in the article, said voids being called programed porosity and differing from the non-programed porosity previously described in the present invention. The programed porosity of the article results from non-sintered or non-melted parts, whereas the non-programed porosity results from the sintered or melted parts.

In one embodiment, the layer manufacturing system for realizing three-dimensional articles through selective laser sintering/melting comprises a high energy source useful for melting or sintering a pulverulent substrate or pulverulent substrate layers.

In one embodiment, the layer manufacturing system for realizing three-dimensional articles through selective laser sintering/melting comprises a laser for melting or sintering a pulverulent substrate or pulverulent substrate layers.

In one embodiment, the layer manufacturing system for realizing three-dimensional article through selective laser sintering/melting comprises a powder tank.

In one embodiment, the layer manufacturing system for realizing three-dimensional article through selective laser sintering/melting comprises a support onto which the article of the present invention is manufactured. In one embodiment, the support is compatible with the pulverulent substrate. "Compatible" means that the support does not taint the device and/or that the support is inert with respect to the manufacturing process, and/or that the support is made from the main substrate and/or that the support presents high compaction. In one embodiment, the support is slightly rough. In another embodiment the support is pre-treated in order that the first layer of pulverulent substrate hooks up onto the support. This pre-treatment may be performed through etching or any other means that a person skilled in the art would find suitable.

In another embodiment, the support may be made from metallic materials, from ceramic materials, from ceramic materials coated with a metallic material or from metallic materials coated with ceramic materials, preferably from ceramic materials.

In one embodiment, the layer manufacturing system for realizing three-dimensional article through selective laser sintering/melting comprises a powder tank filled with a pulverulent substrate comprising at least one main substrate and at least one energy transferring vector.

In one embodiment, the layer manufacturing system for realizing three-dimensional article through selective laser sintering/melting comprises a powder tank filled with a pulverulent substrate comprising at least a ceramics, ceramics in oxide form, metals, metals alloys, bioactive glasses, lead zirconate titanate, silicides, borides, carbides or mixtures thereof; and at least one energy transferring vector comprising carbon, scandium, titanium, vanadium, chromium, manganese, iron, cobalt, nickel, copper, or zinc, or mixture thereof.

In one embodiment, the layer manufacturing system for realizing three-dimensional article through selective laser sintering/melting comprises a powder tank filled with a pulverulent substrate comprising at least a ceramic material in oxide form and at least one energy transferring vector comprising carbon as element.

In one embodiment, the layer manufacturing system for realizing three-dimensional article through selective laser sintering/melting comprises a powder tank filled with a pulverulent substrate comprising at least calcium phosphate such as for instance hydroxyapatite or tricalcium phosphate; and at least one energy transferring vector comprising carbon or silicon carbide.

### Article

The invention also relates to a three-dimensional article and to an article obtainable by the process described hereabove.

In one embodiment, the article has a complex shape.

In one embodiment, the article has a non-programed porosity inferior to 30%, preferably inferior to 20%, preferably inferior to 10%, more preferably inferior to 5%, even more preferably inferior to 2%.

In an embodiment, the article comprises at least 1 ppm, or at least 10 ppm, or at least 100 ppm, or at least 1000ppm of the energy transferring vector.

In a preferred embodiment, the article is used for medical applications.

In one embodiment, the article is a medical device, preferably an implant.

In one embodiment, the article has a shape corresponding to a bone defect.

In one embodiment, the article is to be used for the replacement of a bone defect.

In one embodiment, the shape of the article is patient-specific and obtained through medical imaging.

In one embodiment, the article is use for aeronautical applications.

In one embodiment, the article is use for railway applications.

In one embodiment, the article is use for automotive applications.

In another embodiment the final article is white.

*While various embodiments have been chosen to illustrate the invention, it will be understood by those skilled in the art that some changes and modifications can be made therein without departing from the scope of the invention as defined in the appended claims.*

### DEFINITIONS

In the present invention, the following terms have the following meanings:
- **"Selective laser sintering/melting"** also named in the present invention selective laser sintering/fusion refers to a layer manufacturing technology in which the layers are formed by using an energy source to bond the surface of a bed of powder material in the desired shape.
- **"Layer manufacturing process"** refers to process for manufacturing a part by depositing or bonding successive layers of material. Selective laser sintering/fusion is a kind of layer manufacturing process.
- **"Pulverulent substrate"** refers to the material, in powder form, used in successive layers during the layer manufacturing process.
- **"Main substrate"** refers to a substrate which represents more than 50% by volume of the pulverulent substrate.
- **"Absorption"** refers to the attenuation of the energy of a beam on passage through matter. The dissipated energy here is converted into other forms of energy, e.g. heat.
- **"Energy transferring vector"** refers to a component which can absorb all of, or a major proportion of, radiation in the region from 100 nanometers to 1millimeter; and which can transfer from the radiant energy, thermal energy to its surrounding.
- **"Directed high energy source"** refers to a high energy source, for example a laser, which movement of translation and rotation of the laser beam are predefined and automated.
- **"Layers overlapping"** refers in the present invention to the fact that once a layer of pulverulent substrate is melted of sintered, the melting or sintering process of the subsequent layer may also melt or sinter part of the previous layer. This overlapping depends on the thickness of substrate deposited, the velocity of the energy source and the power of the energy source.
- **"Porosity"** refers to a measure of the void spaces in the biomaterial of the invention, and is measured as a fraction, between 0-1, or as a percentage between 0-100%.
- **"Hatching space"** refers in the present invention to the distance between the scanning lines of the laser beam.

### EXAMPLES

The present invention is further illustrated by the following examples:

### Example 1:

A main substrate of hydroxyapatite, having a granulometry from 5 to 25 µm and a purity above 95% (commercialized by Science Applications Industries) and an energy transferring vector comprising carbon, having a granulometry of 40 nanometers and purity above 97%, are mixed through a wet-process; from 0.1 to 5% by weight of carbon are added to the hydroxyapatite. The mixing is conducted with a laboratory rotary evaporator, called "rotovap", using methanol as a solvent and alumina balls to promote the mixing. The ratio between the powder and the solvent is (1/3)/(2/3). The settings are the following: temperature of 120°C, speed of 25 rpm (revolution per minute) and duration of 24 hours.

The rotary evaporator removes the methanol from de pulverulent substrate by evaporation. By this process, the carbon is well dispersed in the hydroxyapatite powder. The powder is then screened with a mesh size of 50µm to remove larger particles.

The pulverulent substrate comprising hydroxyapatite and carbon is placed in a container of the Phenix^{®} PM100 device commercialized by Phenix System^{®}, so that it can be layered in a plate. The thickness of the powder taken from the container is about 100µm, while the thickness of the resulting layer is about 50µm. The powder is indeed compacted before the sintering/melting process.

The layer is sintered by a Nd-YAG laser beam released from a galvanometric head. The Nd-YAG laser sintered the pulverulent substrate with a power of 40 watts, a velocity of 100millimeter/s and a hatching space of 200µm.

Once the article has been fashioned by selective laser sintering, the article is post-treated to improve the mechanical strength at 1100°C with a heating rate of 10°C/min and a 2 hours-holding time.

### Example 2:

The machine used may be a Phenix® PM100 device commercialized by Phenix Systems®.

A pulverulent substrate comprising a main substrate of tricalcium phosphate having a granulometry from 5 to 25 micrometers and purity above 95% (commercialized by SAI -Science Applications Industries-) and an absorbent agent comprising silicon carbide with a granulometry from 1 nanometer to 100 micrometers and purity above 95% is placed in the powder tank of the Phenix device. The pulverulent substrate is layered with a roll on a plate, where it will be sintered by a laser beam release from a galvanometric head (computer directed optical susceptible to direct a laser beam with high speed and high precision). The thickness of the resulting layer is of about 50 micrometers. A Nd-YAG 100 Watts laser is preferably used to locally impact and sinter the pulverulent substrate. The power of the laser beam may preferably be adjusted to 10% of the total power of the laser; the laser beam may be 10% defocused; the laser deviation may be 80 micrometers; the velocity of the laser beam is of 20 millimeter/s. The trajectory of the laser is defined by the 3D-image. The data of the image (CT scan or IRM for example) are exported in a suitable format, for example DICOM. This file is imported in a software which carries out a partition of the various levels of grey and, starting from various cut-offs, rebuilds the three-dimensional anatomy of the defect. From this 3D file and a computer mediated design software, it is possible to conceive the macrostructure of the implant that fits the defect. The design of the implant is exported in a suitable format (for example format STL, IGES, DXF, HPP, OBJ), and is cut-off in slices corresponding to the thickness of the layers (for example, format SLC). The information for each layer defines the trajectory of the laser.

The trajectory of the laser designs the shape of the 3D-image in the pulverulent substrate, actually in the thickness of the pulverulent substrate. When a layer is processed, the tray supporting the plate is moved down at a distance corresponding to the thickness of a layer and the next layer of pulverulent substrate is layered. The process is repeated until the full biomedical device is fashioned. The laser beams sinters the pulverulent substrate together in the whole thickness of the layer and it action propagates also on the preceding layer, so that the current layer and the preceding layer actually are sintered together.

At the end of the process, the not-sintered residual pulverulent substrate is blown out by any suitable means, preferably mechanical means such as for example micro-aspiration or suction or brushing; then, the biomedical device is recovered. Optionally, before recovering, the biomedical device may be heated to 300-1200°C during 10 minutes to 5 hours.

## Claims

1. A process for manufacturing a three-dimensional article, wherein the process is a selective laser sintering/melting process for manufacturing an article from a pulverulent substrate comprising at least one main substrate and at least one energy transferring vector; said process implementing at least one high energy source.

2. The process for manufacturing a three-dimensional article according to claim **1,** wherein said at least one energy transferring vector comprises as chemical element: carbon, scandium, titanium, vanadium, chromium, manganese, iron, cobalt, nickel, copper, or zinc, or mixture thereof.

3. The process according to claim **2,** wherein the at least one energy transferring vector comprising carbon is carbon or silicon carbide or mixture thereof.

4. The process for manufacturing a three-dimensional article according to anyone of claims **1** to **3,** wherein said at least one main substrate comprises ceramic materials, metals, metals alloys, metals oxide, bioactive glasses, lead zirconate titanate, silicides, carbides, borides, or mixture thereof.

5. The process according to claim **4,** wherein said ceramic materials comprise calcium phosphate such as for example hydroxyapatite or tricalcium phosphate or mixture thereof.

6. The process for manufacturing a three-dimensional article according to anyone of claims **1** to **5,** wherein the process comprises the steps of:
a) Providing a layer of a pulverulent substrate, in a manufacturing chamber,
b) Optionally, controlling the temperature of the manufacturing chamber, or of the walls of the manufacturing chamber,
c) Selective laser sintering/melting of regions of the substrate layer by means of an energy source,
d) Optionally, repeating preceding steps a) to step c) until the desired article has been fashioned layer-by-layer.

7. The process according to anyone of claims **1** to **6,** wherein the amount of energy transferring vector is less than 5% (w/w) relative to the total weight of pulverulent substrate.

8. The process according to anyone of claims **1** to **7,** wherein the particle size of the main substrate ranges from 1 to 500 micrometers, preferably from 5 to 100 micrometers, more preferably from 10 to 25 micrometers.

9. The process according to anyone of claims **1** to **8,** wherein the particle size of the energy transferring vector ranges from 1 nanometer to 500 micrometers, preferably from 1 nanometer to 200 micrometers, more preferably from 10 nanometers to 100 nanometers.

10. The process according to anyone of claims **1** to **9,** wherein the at least one high energy source is a directed high energy source.

11. The process according to anyone of claims **1** to **10,** wherein the at least one high energy source is a laser, preferably a Nd -YAG laser, a CO₂ laser or a Er - YAG laser, more preferably a Nd - YAG laser.

12. A three-dimensional article obtainable by a process according to anyone of claims **1** to **11.**

13. The three-dimensional article of claim **12,** which is a biomedical device.

14. A system for manufacturing the article according to claims **12** or **13,** comprising:
• A computer file storing the description layer by layer of the three-dimensional article to manufacture,
• A directed high energy source for melting or sintering pulverulent substrate or pulverulent substrate layers, the directivity of the high energy source being based on the data of the computer file,
• A powder tank comprising an pulverulent substrate, which is comprising the main substrate and an energy transferring vector; during manufacture of the article, layers of pulverulent substrate from the powder tank are positioned under the high energy source.

15. The system according to claim **14,** wherein the powder tank comprises at least one energy transferring vector comprising as chemical element: carbon, scandium, titanium, vanadium, chromium, manganese, iron, cobalt, nickel, copper, or zinc, or mixture thereof; and at least one main substrate comprising ceramic materials, metals, metals alloys, bioactive glasses, lead zirconate titanate, silicides, borides, carbides or mixture thereof.
